Europäisches Patentamt

**European Patent Office**

Office européen des brevets

(19)

(11) Publication number : **0 437 519 B1**

(12) # EUROPEAN PATENT SPECIFICATION

(45) Date of publication of patent specification :
15.04.92 Bulletin 92/16

(51) Int. Cl.⁵ : **C11C 3/08, A61K 31/23**

(21) Application number : **89911779.0**

(22) Date of filing : **10.10.89**

(86) International application number :
**PCT/DK89/00238**

(87) International publication number :
**WO 90/04012 19.04.90 Gazette 90/09**

(54) **TRIGLYCERIDES AND NUTRITIONAL COMPOSITION COMPRISING SUCH TRIGLYCERIDES.**

(30) Priority : **10.10.88 DK 5652/88**

(43) Date of publication of application :
**24.07.91 Bulletin 91/30**

(45) Publication of the grant of the patent :
**15.04.92 Bulletin 92/16**

(84) Designated Contracting States :
**AT BE CH DE FR GB IT LI LU NL SE**

(56) References cited :
**WO-A-88/09325
US-A- 4 607 052
US-A- 4 701 468
US-A- 4 701 469
US-A- 4 753 963**

(73) Proprietor : **NOVO NORDISK A/S
Novo Allé
DK-2880 Bagsvaerd (DK)**

(72) Inventor : **HANSEN, Tomas, Tage
Tonedraget 5
DK-3450 Alleroed (DK)**
Inventor : **GODTFREDSEN, Sven, Erik
Smedegade 15B
DK-3500 Vaerloese (DK)**

(74) Representative : **Bach, Niels et al
c/o Novo Nordisk A/S Patent Dept. Novo Allé
DK-2880 Bagsvaerd (DK)**

# Description

The invention relates to triglycerides and a nutritional composition comprising such triglycerides.

α-linolenic acid is, together with linoleic acid, the only two essential fatty acids, i.e. the organism cannot synthesize them (Holman, R.T. et al., Nutrition Reviews, 40, 144-47 (1982)). These two fatty acids are the initial precursors for synthesis of prostaglandins. α-linolenic acid for the ω-3 type, linoleic acid for the ω-6 type. It is therefore important that α-linolenic acid is present in any nutritional fat composition. It has been estimated that a daily intake of this fatty acid should be at least 2-10 g. For people on total parenteral nutrition (TPN), it is necessary to have α-linolenic acid in the fat emulsion, or else the person will suffer from essential fatty acid deficiency diseases, which will have serious effects on constitution and fitness.

γ-linolenic acid is a unique fatty acid being a key compound in important physiological events in the body. The compound is generated biosynthetically from linoleic acid by the action of a desaturase enzyme and is further converted enzymatically into arachidonic acid which is the immediate precursor for the prostaglandins. These latter compounds are involved in key physiological events as e.g. the control of the tonus of smooth muscle cells in the blood vessels, and thus blood pressure, or the tonus of the smooth muscle cells in the lungs, and thus the respiratory distress of the asthmatic state.

The first phase in the biological conversion of linoleic acid leads to γ-linolenic acid (GLA). This reaction is controlled by a specific enzyme, Δ-6-unsaturase, which introduces a double bond into the fatty acid chain after carbon atom no. 6. In many cases this conversion does not occur normally and the process becomes a rate limiting step in the synthesis of the ω-6 type of prostaglandins. This is the case for older people, but also people which have various metabolic diseases such as hypoinsulinaemia (insulin dependent diabetes), overproduction of glucagon (a pancreatic hormone antagonistic to insulin), when level of catecholamines is high (stress) and for people which suffer from prolonged fasting. Further trials with GLA have demonstrated a clinical improvement significantly greater than that occurring in response to placebo, on people suffering from atopic eczema, premenstrual breast pain, and rheumatoid arthritis (Horrobin, D.F., Symposium on new aspects of dietary lipids, Göteborg, September 1989). Finally, an increased supply of α-linolenic acid inhibits the production of GLA (Holman, R.T., Fed. Proc., 23, 1062-67 (1964)). As it is difficult to avoid raising the dietary levels of α-linolenic acid by increasing intake of linoleic acid, it can be more effective to increase the intake of GLA directly.

In these and other cases it is important to supply both γ-linolenic and α-linolenic acid not only in high quantities but also in a form which ensures a ready bioavailability of the fatty acids. A particularly important situation arises in connection with treatment of severely ill patients e.g. in a hospital regime. Here parenteral nutrition is often employed as a life saving measure during critical phases of the patient care and, in this connection, fat emulsions play an important role. Thus, emulsions of fat are fed intravenously to patients as a supply of energy. The fats contained in such emulsions are typically based on soy bean oil or other naturally occurring fats which have not been tailored to ensure the most adequate supply of fatty acids nor the most efficient form of the fatty acids in terms of bioavailability.

A very important clinical aspect in regard to the bioavailability is the slower metabolism in comparison to emulsions containing MCT, which are known to cause metabolic acidosis, when infused intravenously.

It is thus the purpose of the present invention to furnish triglycerides which contain γ- and/or α-linolenic acid, which can be incorporated into enteral and parenteral nutritional products, and which provide the essential fatty acid in a highly bio-available form.

The triglycerides according to the invention are 2-linolenoyl-1,3-di(octanoyl/decanoyl) glycerol. It is to be understood that this term encompasses both the two pure 1,3-dioctanoyl triglycerides, the two pure 1,3-didecanoyl triglycerides, and furthermore the two 1-octanoyl-3-decanoyl triglycerides and the two 1-decanoyl-3-octanoyl triglycerides, the 2-position of course in all cases being occupied by α-linolenic acid or γ-linolenic acid. Surprisingly it has been found that these triglycerides exhibit a better absorption of the α-linolenic acid or γ-linolenic acid than the known α-linolenic acid or γ-linolenic acid sources in nutritional compositions.

This invention is a selection invention in the sense that a general chemical formula comprising the triglycerides according to the invention together with a very large number of other triglycerides belong to the prior art, vide FR 2515174, whereas the triglycerides according to the invention do not belong to the prior art. The triglycerides according to the invention are described and synthetized for the first time by the inventors, and also, the superior effect of the triglycerides according to the invention is demonstrated for the first time by the inventors.

Also, a general chemical formula comprising the triglycerides according to the invention appears from US 4,607,052, 4,701,468, 4,701,469, and 4.753,963. Furthermore, it is indicated in these US patents that these triglycerides in general are useful nutrients, which can be administered in an oral, enteral and parenteral way.

A preferred embodiment of the triglycerides according to the invention is characterized by the fact that they have a purity of at least 10%, preferably at least 30%, more preferably at least 50%, even more preferably at least 75%, and most preferably at least 90%. The higher the purity of the triglycerides, the more efficient the absorption of the triglycerides.

Also the invention comprises a nutritional composition, which comprises the triglyceride according to the invention. This composition can be either the triglycerides according to the invention without the other constituents which are necessary for a full nutritional composition, i.e. mainly vitamins, proteins and carbohydrates, or the triglycerides according to the invention together with these other constituents.

A preferred embodiment of the nutritional composition is parenterally administrable. This composition is an emulsion.

A preferred embodiment of the nutritional composition is enterally administrable. This composition is either an emulsion or an oil.

A preferred embodiment of the enteral composition according to the invention is in fluid form. The enteral composition can be an emulsion or a pure oil .

A preferred embodiment of the enteral composition according to the invention is in powder form, whereby the triglycerides are encapsulated or microencapsulated. One of the manners, in which the droplets of triglyceride can be encapsulated, is described in Danochemo Technical Information on microencapsulated Product, Malmparken 5, 2750 Ballerup, Denmark.

## Claims

1. The triglycerides 2-($\alpha$-linolenoyl/$\gamma$-linolenoyl)-1,3-di(octanoyl/decanoyl) glycerol.

2. Triglycerides according to Claim 1, which have a purity of at least 10%, preferably at least 30%, more preferably at least 50%, even more preferably at least 75%, and most preferably at least 90%.

3. Nutritional composition, which comprises the triglycerides according to Claim 1 or 2.

4. Nutritional composition according to Claim 3, which is parenterally administrable.

5. Nutritional composition according to Claim 3, which is enterally administrable.

6. Nutritional composition according to Claim 5, which is in fluid form.

7. Nutritional composition according to Claim 5, which is in powder form, whereby the triglycerides are encapsulated or microencapsulated.

## Patentansprüche

1. Die Triglyceride 2-($\alpha$-Linolenoyl/$\gamma$-Linolenoyl)-1,3-di(octanoyl/decanoyl)glycerin.

2. Triglyceride nach Anspruch 1, dadurch gekennzeichnet, daß sie eine Reinheit von wenigstens 10 %, vorzugsweise wenigtens 30 %, bevorzugter wenigstens 50 %, noch bevorzugter wenigstens 75 % und am bevorzugtesten wenigstens 90 % besitzen.

3. Nahrungszusammensetzung, dadurch gekennzeichnet, daß sie die Triglyceride nach Anspruch 1 oder 2 umfaßt.

4. Nahrungszusammensetzung nach Anspruch 3, dadurch gekennzeichnet, daß sie parenteral verabreichbar ist.

5. Nahrungszusammensetzung nach Anspruch 3, dadurch gekennzeichnet, daß sie enteral verabreichbar ist.

6. Nahrungszusammensetzung nach Anspruch 5, dadurch gekennzeichnet, daß sie in flüssiger Form vorliegt.

7. Nahrungszusammensetzung nach Anspruch 5, dadurch gekennzeichnet, daß sie in Pulverform vorliegt, wobei die Triglyceride gekapselt oder mikrogekapselt sind.

## Revendications

1. Les triglycérides 2-($\alpha$-linolénoyl/$\gamma$-linolénoyl)-1,3-di(octanoyl/décanoyl)glycérol.

2. Triglycérides selon la revendication 1 qui ont une pureté d'au moins 10 %, de préférence d'au moins 30 %, mieux d'au moins 50 %, encore mieux d'au moins 75 %, et tout préférablement d'au moins 90 %.

3. Composition nutritionnelle qui comprend les triglycérides selon la revendication 1 ou 2.

4. Composition nutritionnelle selon la revendication 3 qui peut être administrée par voie parentérale.

5. Composition nutritionnelle selon la revendication 3 qui peut être administrée par voie entérale.

6. Composition nutritionnelle selon la revendication 5 qui est sous une forme fluide.

7. Composition nutritionnelle selon la revendication 5 qui est sous forme d'une poudre, les triglycérides étant encapsulés ou microencapsulés.